# EUROPÄISCHE PATENTSCHRIFT

(11) **EP 0 351 502 B1**
(45) Veröffentlichungstag und Bekanntmachung des Hinweises auf die Patenterteilung: **12.01.1994**
(21) Anmeldenummer: 89107322.3
(22) Anmeldetag: 22.04.1989
(51) Int. Cl.: C12Q 1/02, C12P 13/04, C12N 15/01

(54) **Verfahren zur Auswahl produktiver, Aminosäuren ausscheidender Stämme von Mikroorganismen**
Method for screening productive micro-organism strains which secrete amino acids
Procédé pour le criblage de souches microbienne productives, sécrétant des acides aminés

(30) Priorität: 22.07.1988 DE 3824937
(43) Veröffentlichungstag der Anmeldung: 24.01.1990
(73) Patentinhaber: Degussa Aktiengesellschaft, 60311 Frankfurt (DE)
(72) Erfinder: Kircher, Manfred, Dr., D-4800 Bielefeld 12 (DE); Aragòn, Julio Cesar, Dr., D-4800 Bielefeld 1 (DE); Bachmann, Bernd, Dr., D-4806 Werther (DE); Grosse Wiesmann, Joachim, Dr., D-4800 Bielefeld 13 (DE)

(56) Entgegenhaltungen:
- US-A- 4 275 157

## Beschreibung

Die Erfindung betrifft ein Verfahren zur Auswahl (Screening) produktiver, Aminosäuren ausscheidender Stämme von Mikroorganismen für den Einsatz bei der Fed-Batch-Fermentation.

Es ist generell anwendbar und wird im folgenden Text anhand der Auswahl von Lysin produzierenden Stämmen der Gattung Corynebacterium glutamicum demonstriert.

Die essentielle Aminosäure-L-Lysin ist als Nahrungs- und Tierfutterzusatz sowie als Wirkstoff und Bestandteil von pharmazeutischen Produkten von großer industrieller Bedeutung.
Für die Herstellung von L-Lysin ist die Fed-Batch-Fermentation das bedeutendste Verfahren. Vor allem coryneforme Bakterien der Gattungen Corynebacterium und Brevibacterium werden in der Produktion eingesetzt. Durch Mutationen ist die Regulation der Lysin-Biosynthese dieser Stämme so verändert, daß sie Lysin über den Eigenbedarf hinaus produzieren und in das Medium ausscheiden.

Derartige überproduzenten erhält man durch Suche nach Mutanten, in denen einzelne Schritte des Aminosäurestoffwechsels blockiert sind (z. B. Hse- oder Thr-Auxotrophe), die gegen ein oder mehrere Analoga von Lysin resistent sind oder die weitere Mutationen enthalten. Hochleistungsstämme besitzen mehrere Auxotrophien, Analoga-Resistenzen oder eine Kombination von Mutationen. Eine zusammenfassende Darstellung der genetischen Methoden zur Entwicklung von Lysin-Produzenten geben 0. Tosaka und K. Takinami (Prog. Ind. Microbiol. (Biotechnol. Amino Acids) 24, (1986), 152-172).

Die Suche nach stämmen, die Aminosäuren produzieren, wird als Screening bezeichnet. Im Screening werden in einem Ausgangsstamm mittels gebräuchlicher chemischer oder physikalischer Mutagene (z. B. MNNG oder UV) zufällige Mutationen induziert und mit üblichen mikrobiologischen Methoden Mutanten selektioniert. Da nicht alle selektierten Mutanten Aminosäureproduzenten sind, schließtsich ein Test auf das Vorhandensein der gewünschten Aminosäure an.
Dieser Test wird üblicherweise submers in Schüttelkolben (10 ml - 2000 ml) durchgeführt, wobei die Organismen zum Wachstum und zur Produktion eine sterile Nährlösung (5 - 50 ml) benötigen, die eine Kohlenstoff- und Energiequelle (z. B. Glucose, Melasse) sowie eine Stickstoffquelle (z. B. Ammonium-Sulfat), Vitamine (z. .B. Biotin, Thiamin) und verschiedene Salze enthält. Die Fermentation wird bei einer Temperatur zwischen 20 °C und 40 °C aerob unter Schütteln (50 - 400rpm) durchgeführt . Der pH-Wert der Nährlösung beträgt 5 - 9. Die Schüttelkolben werden nach 72 h bis 96 h geerntet und die jeweilige Aminosäure-Konzentration im Medium wird mit einer üblichen analytischen Methode bestimmt. Die nach dieser Zeit im Kolben erreichte Konzentration dient als Kriterium für die Bewertung der getesteten Mutante. Die Vorgehensweise ist in zahlreichen Patenten, z. B. US-PS 3,707,441 US-PS 3,732,144, US-PS 3,708,395, US-PS 4,275,157 und in Publikationen z. B. K. Sano und I. Shiio (1967),
J. Gen. Appl. Microbiol. 13, 349-358 und (1970)
J. Gen. Appl. Microbiol. 16, 373-391 und (1971)
J. Gen. Appl. Microbiol. 17, 97 -113),
0. Tosaka, K. Takinami und Y.Hirose (1978)
Agric. Biol. Chem. 42, 745-752) und
M. Schonfeldt und T. Watson (1982) South African Food Rev. 9, 111-112) beschrieben.
Der Klon mit der höchsten Konzentration wird als der Beste beurteilt.

Der Erfolg eines solchen Screenings hängt entscheidend davon ab, ob unter der Vielzahl induzierter Mutanten die bestgeeignetste erkannt wird. Für die Beurteilung ist nach dem Stand der Technik der Schüttelkolbentest das entscheidende Raster. Dabei dient, wie oben erwähnt, die nach 72 bis 96 h im Medium angereicherte Konzentration der Aminosäure üblicherweise als Kriterium. Zu diesem Zeitpunkt ist nach unseren Ergebnissen die Produktion im Schüttelkolben abgeschlossen
(K. Nakayama in: Comprehensive Biotechnology 3, 697 . 620; ed M. Moo-Young: Pergamon Press (1985)). Ausgewählt werden dann Stämme, die unter den im Schüttelkolben vorgegebenen Bedingungen die größte Menge der gewünschten Aminosäure ausscheiden.

Dieses Screening-Verfahren wird allgemein verwendet, da es eine relativ schnelle Auswahl unter den jeweils in einer Vielzahl zur Verfügung stehenden Mutanten ermöglicht und verfahrenstechnisch einfach durchzuführen ist.

Ein anderes Verfahren, wie die Bestimmung der Wachstumsrate während der Fermentation, erschien dem Fachmann bei der großen Zahl der zu untersuchenden Stämme als zu aufwendig und auch nicht unbedingt aussagekräftig für die Aminosäureproduktivität eines Stammes.

Obwohl in der Regel die Auswahl der Produktionsstämme nach dem oben beschriebenen Verfahren erfolgt, ist ein bestimmter Nachteil nicht zu vernachlässigen.

Man stellt immer wieder fest, daß sich nach dem bekannten Screening-Verfahren ausgewählte Stämme, die dieselben oder vergleichbare Umsätze zeigten, bei der Verwendung in einem Fed-Batch-Prozeß deutlich unterscheiden und zu wesentlich voneinander abweichenden Aminosäurekonzentrationen in dem Kulturmedium führen.

Gegenstand der Erfindung ist ein Verfahren zur Auswahl produktiver, Aminosäuren ausscheidender Stämme von Mikroorganismen in einem mit den betreffenden Mikroorganismen beimpften Kulturmedium, für den Einsate bei der Fed-Batch-Fermentation dadurch gekennzeichnet, daß man zwischen Beginn und Endpunkt der Produktionsphase die Konzentration der gewünschten Aminosäure mindestens einmal bestimmt und die Mutante auswählt, die bis zu diesem(n) Zeitpunkt(en) die größte Menge dieser Aminosäure ausgeschieden hat.

Führt man nur eine Messung während der Produktionsphase durch, wählt man als Zeitpunkt im allgemeinen den Ablauf der Hälfte der Fermentationsdauer.

Für das erfindungsgemäße Verfahren kommen alle Mikroorganismen in Frage, die Aminosäuren ausscheiden, insbesondere der Gattungen Corynebacterium und Brevibacterium.

Beispielhaft nachgewiesen wird die überlegenheit des erfindungsgemäßen Verfahrens anhand von L-Lysin produzierenden Mikroorganismen der Gattung Corynebacterium glutamicum.
Die Bezeichnung Fed-Batch-Verfahren entspricht einer geläufigen Terminologie und bezeichnet einen Fermentationsprozeß, bei dem während der Fermentation Nährstoffe, wie z. B. Kohlenstoffquellen und Stickstoffquellen nachgefüttert werden.

Wie die Beispiele zeigen, werden bei dem erfindungsgemäßen Verfahren andere stämme als nach dem üblichen Schüttelkolbentest ausgewählt.

Zur Bestimmung der L-Lys-Anreicherung während der Produktionsphase wurde die L-Lys-Konzentration nach 24 h Schüttelinkubation, zur Bestimmung der L-Lys-Endkonzentration nach Abschluß der Produktion nach 48 h bestimmt. Die Beispiele beziehen sich auf stämme von Corynebacterium glutamicum.

### Beispiele

### Beispiel 1: Schüttelkolbentest DM 180-1

Stamm DM 180-1 wird vom Stammröhrchen in 4 ml Caco-Bouillon geimpft, 16 h bei 30°C unter Schütteln bebrütet und anschließend nach 1 :3-Verdünnung in frische Caso-Bouillon (4 h, 30 °C; Schütteln) in einen 100 ml Erlenmeyerkolben mit Schikane, der das Testmedium (9 ml) enthält, inokuliert (1 ml). Das Testmedium enthält 240 g/l Melasse, 100 ml/l Sojamehl-Hydrolysat, 12 g/l Ammoniumsulfat und 10 g/l Calciumcarbonat. Nach 24 h Schütteln (300 rpm) bei 30 °C enthält das Medium 19,0 g/l L-Lys x HCl, nach 48 h 32,2 g/l L-Lys x HCl.

### Beispiel 2: Schüttelkolbentest DM 290-2

Stamm DM 290-2 wird, wie in Beispiel 1 beschrieben, angezogen und im Schüttelkolben inkubiert. Nach 24 h enthält das Medium 18,1 g/l L-Lys x HCl, nach 48 h 34,4 g/l L-Lys x HCl.

### Beispiel 3: Schüttelkolbentest DM 282-2

Stamm DM 282-2 wird, wie in Beispiel 1 beschrieben, angezogen und im Schüttelkolben inkubiert. Nach 24 h enthält das Medium 24,9 g/l L-Lys x HCl, nach 48 h 30,1 g/l L-Lys x HCl.

Die Ergebnisse der Beispiele 1 - 3 sind in Tabelle 1 zusammengefaßt:

**Tabelle 1**

| L-Lysin-Ausscheidung im Schüttelkolben | | |
|---|---|---|
| Stamm | L-Lys x HCl (g/l) | |
| | 24 h | 48 h |
| DM 180-1 | 19,0 | 32,2 |
| DM 290-2 | 18,1 | 34,4 |
| DM 282-2 | 24,9 | 30,1 |

Die statistische Auswertung dieser Daten mit Studentt-Test belegt, daß sich die L-Lysin-Ausscheidung der drei Stämme nach 24 h und 48 h hochsignifikant voneinander unterscheidet.

Nach dem herkömmlichen Verfahren wählt man unter diesen stämmen denjenigen mit der höchsten Endkonzentration, also DM 290-2, nach dem erfindungsgemäßen Verfahren würde DM 282-2 als mit der höchsten L-Lysin-Konzentration nach 24 h ausgewählt werden.

Wie sich die Stämme in der Fed-Batch-Fermentation verhalten, zeigen die Beispiele 4 - 6.

### Beispiel 4: Fed-Batch-Fermentation DM 180-1

0,1 l Caso-Bouillon werden im 2 l-Erlenmeyerkolben mit Schikane angeimpft und schüttelinkubiert (30 °C, 24 h, 150 rpm). Mit dieser Zellsuspension wird ein Vorfermenter mit 8 Medium M1 (60 g/l Melasse, 10 g/l Ammoniumsulfat, 120 ml/l Sojamehlhydrolysat mit 1 ml/l Phosphorsäure) beimpft und nach 9 h Bebrütung (33 ⁰C) unter Rühren und Belüftung werden 1,3 l Zellsuspension in den Hauptfermenter überführt (13 l Medium M1). Nach 6 h (33 °C, 800 rpm, pH 7,0, 1,5 vvm Belüftung) wird mit der Zugabe (200 g/h) von Medium M2 begonnen (1245 g/l Melasse, 34 g/l Ammoniumsulfat; 30 °C, rpm/vvm = f (pO₂ = 30 %); pH 7,5).
Nach 72 h werden 41 g/l L-Lys x HCl angereichert.

### Beispiel 5: Fed-Batch-Fermentation DM 290-2

0,4 l Caso-Bouillon werden im 2 l-Erlenmeyerkolben mit Schikane angeimpft und schüttelinkubiert (30 °, 13 h, 150 rpm). Mit dieser Zellsuspension wird ein Vorfermenter (10 l Medium Caso-Bouillon mit 7,5 g/l Saccharose) beimpft und 12 h unter Rühren und Belüftung inkubiert (30 ⁰C). 1,4 l Zellsuspension werden in einen weiteren Vorfermenter mit 14 l Medium M1 (60 g/l Melasse, 10 g/l Ammoniumsulfat, 120 ml/l Sojamehlhydrolysat mit 1 ml/l Phosphorsäure) überführt und nach 9 h Bebrütung (33 °C) unter Rühren und Belüftung werden 2,31 l Zellsuspension in den Hauptfermenter geimpft (14,4 l Medium M1). Nach 6 h (33 °C, 800 rpm, pH 7,0, 1,5 vvm Belüftung) wird mit der Zugabe (200 g/h) von Medium M2 begonnen.
(1100 g/l Melasse, 22 g/l Ammoniumsulfat; 30 °C, rpm/vvm = f (pO₂ = 30 %); pH 7,5).

Nach 72 h enthält das Medium 37,5 g/l-L-Lys x HCl.

### Beispiel 6: Fed-Batch-Fermentation DM 282-2

0,4 l Caso-Bouillon werden im 2 l-Erlenmeyerkolben mit Schikane angeimpft und schüttelinkubiert (30 °C, 10 h, 150 rpm). Mit dieser Zellsuspension wird ein Vorfermenter (8l Medium M1 (60 g/l Melasse, 10 g/l Ammoniumsulfat, 120 ml/l Sojamehlhydrolysat mit 1 ml/l Phosphorsäure) beimpft und nach 9 h Bebrütung (330 °C) unter Rühren und Belüftung werden 21 Zellsuspensionen in den Hauptfermenter überführt (10l Medium M1). Nach 6 h (33 °C, 800 rpm, pH 7,0, 1,5 vvm Belüftung) wird mit der Zugabe (200 g/h von Medium M2 begonnen (1245 g/l Melasse, 34 g/l Ammoniumsulfat; 30 °C, rpm/vvm = f (pO₂ = 30 %); pH 7,5).
Nach 72 h enthält das Medium 76 g/l L-Lys x HCl.

Die Ergebnisse der Beispiele 4 - 6 sind in Tabelle 2 zusammengefaßt:

**Tabelle 2**

| L-Lysin-Ausscheidung im Fermenter (Fed-Batch) | |
|---|---|
| Stamm | L-Lys x HCl (g/l) 72 h |
| DM 180-1 | 41 |
| DM 290-2 | 37,5 |
| DM 282-2 | 76 |

Der nach dem erfindungsgemäßen Kriterium der Produktionsgeschwindigkeit ausgewählte Stamm DM 282-2 ist im Fed-Batch-Prozeß dem nach dem herkömmlichen Kriterium Endkonzentration selektierten überlegen.

## Patentansprüche

1. Verfahren zur Auswahl produktiver` Aminosäuren ausscheidender Stämme von Mikroorganismen in einem mit den betreffenden Mikroorganismen beimpften Kulturmedium für den Einsatz bei Fed-Batch-Fermentationen,
dadurch gekennzeichnet, daß man zwischen Beginn und Endpunkt der Produktionsphase die Konzentration der gewünschten Aminosäure mindestens einmal bestimmt und den Stamm auswählt, die bis zu diesem(n) Zeitpunkt(en) die größte Menge dieser Aminosäure ausgeschieden hat.

2. Verfahren gemäß Anspruch 1
dadurch gekennzeichnet daß man bei nur einmaliger Bestimmung der Konzentration als Zeitpunkt für diese Messung den Ablauf der Hälfte der Fermentationsdauer wählt.

## Claims

1. A process for the selection of productive, amino acid-excreting strains of microorganisms in a culture medium inoculated with the given microorganisms for use in feed batch fermentations, characterised in that the concentration of the desired amino acid is determined at least once between the onset and the end point of the production phase and that strain is selected which has excreted the largest quantity of this amino acid up to this or these point(s) in time.

2. A process according to Claim 1, characterised in that when the concentration is determined only once, the point in time chosen for this measurement is the expiry of half the fermentation time.

## Revendications

1. Procédé de sélection de souches de microroganismes productifs sécrétant des acides aminés dans un milieu de culture inoculé avec les microorganismes correspondants pour l'utilisation dans des fermentations fed-batch,
caractérisé :
- en ce qu'on détermine au moins une fois entre le début et la fin de la phase de production la concentration de l'acide aminé désiré et on choisit la souche, qui a secrété jusqu'à ce (ces) moment(s) la plus grande quantité de cet acide aminé.

2. Procédé selon la revendication 1,
caractérisé :
- en ce que dans le cas d'une seule détermination de la concentration, on choisit comme instant, pour cette mesure, le déroulement à la moitié de la durée de fermentation.
